(19) Europäisches Patentamt
European Patent Office
Office européen des brevets

(11) **EP 4 311 823 A1**

(12) **EUROPEAN PATENT APPLICATION**
published in accordance with Art. 153(4) EPC

(43) Date of publication:
**31.01.2024 Bulletin 2024/05**

(21) Application number: **22913659.3**

(22) Date of filing: **13.10.2022**

(51) International Patent Classification (IPC):
*C07C 31/18* $^{(2006.01)}$ *C07C 29/78* $^{(2006.01)}$
*C07C 29/76* $^{(2006.01)}$ *C09B 61/00* $^{(2006.01)}$
*B01J 19/00* $^{(2006.01)}$

(52) Cooperative Patent Classification (CPC):
**C09B 67/006; C07H 1/00**

(86) International application number:
**PCT/CN2022/125220**

(87) International publication number:
**WO 2023/124395 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.12.2021 CN 202111644482**

(71) Applicant: **Zhejiang Huakang Pharmaceutical Co., Ltd.**
**Quzhou, Zhejiang 324302 (CN)**

(72) Inventors:
• **LI, Mian**
  **Santa Clara, California 95054 (US)**

• **XU, Weidong**
  **Quzhou, Zhejiang 324302 (CN)**
• **CHEN, Deshui**
  **Quzhou, Zhejiang 324302 (CN)**
• **CHENG, Xinping**
  **Quzhou, Zhejiang 324302 (CN)**
• **LIAO, Chengjun**
  **Quzhou, Zhejiang 324302 (CN)**
• **WU, Qiang**
  **Quzhou, Zhejiang 342302 (CN)**
• **YANG, Wulong**
  **Quzhou, Zhejiang 342302 (CN)**
• **QIN, Shufang**
  **Quzhou, Zhejiang 342302 (CN)**

(74) Representative: **Sun, Yiming**
**HUASUN Patent- und Rechtsanwälte**
**Friedrichstraße 33**
**80801 München (DE)**

(54) **SYSTEM AND METHOD FOR CO-PRODUCING XYLITOL AND CARAMEL COLOR BY USING XYLOSE MOTHER LIQUOR**

(57) The present disclosure discloses a system for co-producing a xylitol and a caramel pigment by utilizing a xylose mother liquid. The system includes a raw material tank, a filter, a nanofiltration membrane device, a first ion exchange device, a chromatographic separation device, a refined hydrogenation assembly, and a browning reaction assembly that are sequentially connected through a pipeline. The chromatographic separation device is configured to obtain an extracted liquid and a raffinate liquid by separating the xylose mother liquid that flows through the chromatographic separation device. The refined hydrogenation assembly is configured to prepare a crystal xylitol by performing a refined hydrogenation processing on the extracted liquid. The browning reaction assembly is configured to prepare the caramel pigment by performing a browning reaction processing on the raffinate liquid. The present disclosure also discloses a method for co-producing a xylitol and a caramel pigment by utilizing a xylose mother liquid. The present disclosure prepares the crystal xylitol by purifying and crystallizing the xylose mother liquid and preparing the caramel pigment through the raffinate liquid obtained after a chromatographic separation, which improves the value of the xylose mother liquid.

EP 4 311 823 A1

Fig 2

2

**Description**

**TECHNICAL FIELD**

The present disclosure relates to the field of utilization of a xylose mother liquid, and in particular, to a system and method for producing a xylitol and a caramel pigment by utilizing a xylose mother liquid.

**BACKGROUND**

The production of xylitol is mainly based on raw materials such as corn cobs and corn stalks. Xylose is extracted from the raw materials, and then the xylose is hydrogenated to produce the xylitol. However, a xylose component content in the xylose mother liquid after the xylose is extracted is still very high. If the xylose component in the xylose mother liquid is extracted again to prepare the xylitol, and the raffinate liquid obtained after a chromatographic separation is used to prepare caramel pigment, it will be more beneficial to the utilization of resources and energy. Chinese Patent Application No. CN109503676A introduces a method for preparing a xylitol and a mixed syrup from a xylose mother liquid, which obtains a crystal xylitol and the mixed syrup through processes such as pre-processing, chromatographic separation, activated carbon decolorization, ion exchange desalination, evaporation and crystallization, and hydrogenation refinement. However, the mixed syrup is not reused, which reduces the value of the xylose mother liquid.

**SUMMARY**

The present disclosure provides a system and method for co-producing a xylitol and a caramel pigment by utilizing a xylitol mother liquid. The system and method adopt a nanofiltration membrane device for separation and decolorization and an ion exchange device for desalination, which improves a yield rate of the xylitol. Besides, by reutilizing a raffinate liquid obtained from a chromatographic separation, the caramel pigment can be obtained as an additional product, thereby increasing a utilization value of the xylitol mother liquid.

The present disclosure provides the system for producing a xylitol and a caramel pigment by utilizing a xylose mother liquid. The system may include a raw material tank, a filter, a nanofiltration membrane device, a first ion exchange device, a chromatographic separation device, a refined hydrogenation assembly, and a browning reaction assembly that are sequentially connected tough a pipeline. The raw material tank may be configured to store the xylitol mother liquid. The filter may be configured to filter impurities in the xylitol mother liquid. The nanofiltration membrane device may be configured to decolorize the xylitol mother liquid that flows through the nanofiltration membrane device and obtain a retentate liquid and a permeation liquid respectively. The retentate liquid is a pigment liquid and the permeation liquid is a decolorized liquid. The first ion exchange device may be configured to desalt the decolorized liquid that flows through the first ion exchange device to obtain an ion exchange liquid. The first ion exchange device may be configured to separate an extracted liquid with a high xylose component content and a raffinate liquid with a low xylose component content from the ion exchange liquid that flows through the first ion exchange device. The refined hydrogenation assembly may be configured to prepare a crystal xylitol by performing a refined hydrogenation processing on the extracted liquid. The browning reaction assembly may be configured to prepare the caramel pigment by performing a browning reaction processing on the raffinate liquid.

Furthermore, the refined hydrogenation assembly may include an evaporation and concentration device, a crystallization tank, a crystal xylose storage tank, a dissolving tank, and a hydrogenation reactor, a second ion exchange device, and a vacuum crystallization assembly. The evaporation and concentration device may be configured to further concentrate the extracted liquid. The crystallization tank may be configured to crystallize a xylose. The crystal xylose storage tank may be configured to store the crystal xylose. The dissolving tank may be configured to dissolve the crystal xylose into a liquid and store a xylose liquid. The hydrogenation reactor may be configured to perform a hydrogenation and reduction reaction on the xylose liquid to generate the xylitol. The second ion exchange device may be configured to remove anions and cations in a xylitol liquid. The vacuum crystallization assembly may be configured to crystallize the xylitol liquid processed by the ion exchange device to obtain the crystal xylitol.

Furthermore, the browning reaction assembly may include a concentration tank, a browning reaction reactor, and a browning reaction filter. The concentration tank may be configured to concentrate the raffinate liquid to a certain concentration and store a concentrated raffinate liquid. The browning reaction reactor may be configured to prepare the caramel pigment by performing the browning reaction processing on the raffinate liquid. The browning reaction filter may be configured to filter solid impurities in the caramel pigment.

The present disclosure provides a method for co-producing a xylitol and a caramel pigment by utilizing a xylose mother liquid, the method comprising:

in step one, transporting a raw material of the xylose mother liquid in a raw material tank may to a filter through a pipeline for filtering impurities, and then transporting the raw material of the xylose mother liquid to a nanofiltration membrane device for a decolorizing processing;

in step two, transporting the raw material of the xylose mother liquid that is filtered and decolorized to a first ion exchange device for a desalting processing and obtaining an ion exchange liquid; and

in step three, transporting the ion exchange liquid to a chromatographic separation device for a chromatographic separation processing, obtaining an extracted liquid with a high xylose component content and a raffinate liquid with a low xylose component content after the chromatographic separation processing, transporting the extracted liquid to a refined hydrogenation assembly for the refined hydrogenation processing to obtain a crystal xylitol with a purity over 99%, and transporting the raffinate liquid to a browning reaction assembly for the browning reaction processing to prepare the caramel pigment.

Furthermore, in the step one, a mass percentage concentration of a dry matter of the xylose mother liquid may be 50 to 60wt%. The mass percentage concentration of the dry matter may be a sugar concentration. In the dry matter, a content of glucose may be 12 to 18wt%, a content of xylose may be 40 to 50wt%, a content of arabinose may be 17 to 23wt%, a content of mannose may be 10 to 22wt%, and a content of galactose may be 0 to 6wt%.

Furthermore, during the decolorizing processing in the step one, an operating temperature of the nanofiltration membrane device may be 40 °C to 48 °C, an operating pressure of the nanofiltration membrane device may be 25 bar to 35 bar, and a yield rate may reach 90% to 98%.

Furthermore, during the ion exchange desalting processing in the step two, an electrical conductivity rate may be controlled being smaller than 50 us/cm, and a yield rate may reach 90% to 98 %.

Furthermore, in the step three, the xylose may be dissolved in water. A refraction may be controlled between 50% to 60% and a pH value may be 5.00 to 7.00. A nickel catalyst with a mass percentage of 0.01 % to 0.02% may be added. A reaction temperature may be controlled between 130°C to 140°C, a steam pressure may be above 0.4 MPa, a hydrogenation reaction pressure may be controlled between 7.0 MPa to 9.5 MPa, and a hydrogenation reaction time may be 60 minutes to 120 minutes. The raffinate liquid may be concentrated to a refraction between 75% to 85% and a pH value may be adjusted between 7.00 to 9.00. A compounded amino compound with a mass percentage of 6% to 12% (compounded by a urea and an ammonium carbonate with a compounding ratio of 1:2 to 2:1) may be added as a catalyst, and a reaction temperature of the browning reaction may be controlled being between 120°C to 140 °C and a time of the browning reaction may be 60 minutes to 240 minutes.

Furthermore, the refined hydrogenation processing of the refined hydrogenation assembly in the step three may include: transporting the extracted liquid after evaporation and concentration to the crystallization tank, dissolving the crystal xylose in water, and transporting the xylose liquid into the hydrogenation reactor for a hydrogenation reaction to obtain a xylitol solution, settling the xylitol solution after the hydrogenation reaction is completed to remove the catalyst, performing the desalting processing on a supernatant liquid of the settling by the second ion exchange device, transporting the desalted liquid to the vacuum crystallization assembly for vacuum evaporation and concentration and vacuum boiling of sugar and crystallization to precipitate the crystal xylitol, and obtaining the crystal xylitol through a centrifugation operation and a drying operation.

Furthermore, the browning reaction processing of the browning reaction assembly in the step three may include: obtaining a caramel pigment liquid by performing a concentration processing, the browning reaction processing, and a filtering processing on the raffinate liquid. A red index of the caramel pigment liquid may be over 7, and an absorbance of the caramel pigment liquid at 610 nm may be over 0.07.

Compared to existing technologies, the system and method for co-producing a xylitol and a caramel pigment by utilizing a xylose mother liquid in the present disclosure has following features:

1. The present disclosure uses a nanofiltration membrane separation technology to decolorize the xylose mother liquid, thereby improving a production yield.

2. The caramel pigment product can be obtained by performing the concentration processing, the browning reaction processing, and the filtering processing on the raffinate liquid. The color rate of the caramel pigment product can reach 20000EBC, and the red index of the caramel pigment product can reach 7.

3. By preparing the caramel pigment using the raffinate liquid is prepared to prepare utilization rate of a reducing sugar can be over 70%, and the value of the raffinate liquid can be doubled.

**BRIEF DESCRIPTION OF THE DRAWINGS**

FIG. 1 is a schematic diagram illustrating an exemplary system for co-producing a xylitol and a caramel pigment by utilizing a xylose mother liquid according to the present disclosure.

FIG. 2 is a flow chart illustrating an exemplary process for co-producing the xylitol and the caramel pigment by utilizing the xylose mother liquid according to the present disclosure.

**DETAILED DESCRIPTION**

In order to make technical problems to be solved, technical solutions and beneficial effects of the present disclosure clearer, the following detailed description is provided in conjunction with the accompanying drawings and embodiments. It should be understood that specific embodiments described herein are only used to explain the present disclosure and are not intended to limit the present disclosure.

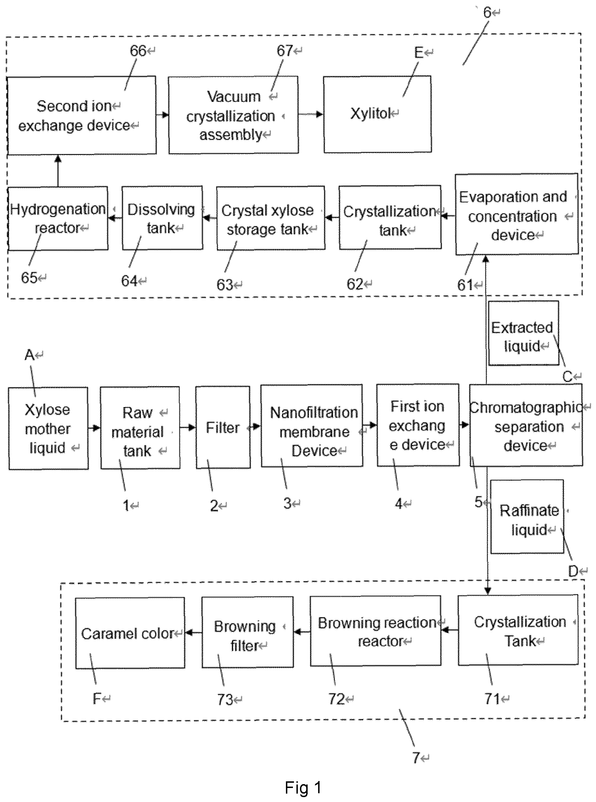

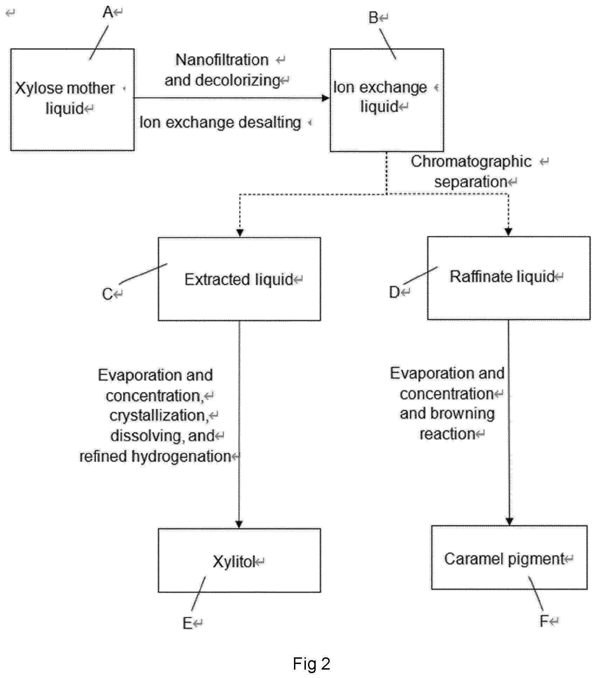

Referring to FIG. 1 and FIG. 2, the present disclosure discloses a system for co-producing a xylitol and a caramel pigment by utilizing a xylose mother liquid. The system may include a raw material tank 1, a filter 2, a nanofiltration membrane device 3, a first ion exchange device 4, a chromatographic separation device 5, a refined hydrogenation assembly 6, and a browning reaction assembly 7 that are sequentially connected through a pipeline.

The raw material tank 1 may be configured to store a xylose mother liquid A. The xylose mother liquid A may be a by-product obtained from extracting a xylose component from a biomass raw material while preparing the xylitol. In a dry matter of the xylose mother liquid A, a content of glucose may be 12 to 18wt%, a content of xylose may be 40 to 50wt%, a content of arabinose may be 17 to 23wt%, a content of mannose may be 10 to 22wt%, and a content of galactose may be 0 to 6wt%.

The filter 2 may be configured to filter impurities in the xylose mother liquid A. The nanofiltration membrane device 3 may be configured to decolorize the xylose mother liquid A that flows through the nanofiltration membrane device 3 to obtain a retentate liquid and a permeation liquid. The retentate liquid is a pigment liquid and the permeation liquid is a decolorized liquid. The first ion exchange device 4 may be configured to desalt the decolorized liquid to obtain an ion exchanged liquid B, and the chromatographic separation device 5 may be configured to separate the ion exchanged liquid B that flows through the chromatographic separation device 5 to obtain an extracted liquid C with a high xylose component content and a raffinate liquid D with a low xylose component content. The refined hydrogenation assembly 6 may be configured to perform a refined hydrogeneration processing on the extracted liquid C to prepare a crystal xylitol E, and the browning reaction assembly 7 may be is configured to perform a browning reaction processing on the raffinate liquid D to prepare a caramel pigment F.

Specifically, the refined hydrogenation assembly 6 may include an evaporation and concentration device 61, a crystallization tank 62, a crystal xylose storage tank 63, a dissolving tank 64, and a hydrogenation reactor 65, a second ion exchange device 66, and a vacuum crystallization assembly 67. The evaporation and concentration device 61 may be configured to further concentrate the extracted liquid C, the crystallization tank 62 may be configured to crystallize a xylose, the crystal xylose storage tank 63 may be configured to store the crystal xylose, the dissolving tank 64 may be configured to dissolve the crystal xylose into a liquid and store a xylose liquid, the hydrogenation reactor 65 may be configured to perform a hydrogenation and reduction reaction on the xylose liquid to generate the xylitol E, the second ion exchange device 66 may be configured to remove anions and cations in a xylitol E liquid, and the vacuum crystallization assembly may be configured to perform a crystallization processing on the xylitol E liquid processed by the ion exchange device to obtain the crystal xylitol E.

Furthermore, the browning reaction assembly 7 may include a concentration tank 71, a browning reaction reactor 72, and a browning reaction filter 73. The concentration tank 71 may be configured to concentrate the raffinate liquid D to a certain concentration and store a concentrated raffinate liquid, the browning reaction reactor 72 may be configured to prepare the caramel pigment F by performing the browning reaction processing on the raffinate liquid D, and the browning reaction filter 73 may be configured to filter solid impurities in the caramel pigment F.

The present disclosure further discloses a method for co-producing a xylitol and a caramel pigment by utilizing a xylitol mother liquid, including following steps:

in step one, transporting a raw material of the xylose mother liquid A in the raw material tank 1 through a pipeline to the filter 2 for filtering impurities, and then transporting the raw material of the xylose mother liquid A to the nanofiltration membrane device 3 for a decolorizing processing;

in step two, transporting the raw material of the xylose mother liquid A that is filtered and decolorized to the first ion exchange device 4 for a desalting processing and obtaining the ion exchange liquid B; and

in step three, transporting the ion exchange liquid B to the chromatographic separation device 5 for a chromatographic separation processing, obtaining the extracted liquid C with a high xylose component content and the raffinate liquid D with a low xylose component content after the chromatographic separation processing, transporting the extracted liquid C to the refined hydrogenation assembly 6 for the refined hydrogenation processing to obtain the crystal xylitol E with a purity over 99%, and transporting the raffinate liquid D to the browning reaction assembly 7 for the browning reaction processing to prepare the caramel pigment F.

Specifically, during the decolorizing processing in the step one, an operating temperature of the nanofiltration membrane device 3 may be 40 °C to 48 °C, an operating pressure of the nanofiltration membrane device 3 may be 25 bar to 35 bar, and a yield rate may reach 90% to 98%.

Specifically, during the ion exchange desalting processing in the step two, an electrical conductivity rate may be controlled being smaller than 50 us/cm, and a yield rate may reach 90% to 98 %.

Specifically, in the step three, the xylose may be dissolved in water. A refraction may be controlled between 50% to 60% and a pH value may be 5.00 to 7.00. A nickel catalyst with a mass percentage of 0.01% to 0.02% may be added. A reaction temperature may be controlled between 130 °C to 140°C, a steam pressure is above 0.4 MPa, a hydrogenation reaction pressure may be controlled between 7.0 MPa to 9.5 MPa, and a hydrogenation reaction time may be 60 minutes to 120 minutes. The raffinate liquid D may be concentrated to a refraction between 75% to 85% and a pH value may be adjusted between 7.00 to 9.00. A compounded amino compound with a mass percentage of 6% to 12% (compounded by a urea and an ammonium carbonate with a compounding ratio of 1:2 to 2:1) may be added as a catalyst, and a reaction temperature of the browning reaction may be controlled being between 120°C to 140 °C and a time of the browning reaction may be 60 minutes to 240 minutes.

The refined hydrogenation processing of the refined hydrogenation assembly 6 in the step three may include: transporting the extracted liquid C after evaporation and concentration to the crystallization tank 62, dissolving the crystal xylose in water, and transporting the xylose liquid into the hydrogenation reactor 65 for a hydrogenation reaction to obtain a xylitol solution, settling the xylitol solution after the hydrogenation reaction is completed to remove the catalyst, performing the desalting processing on a supernatant liquid of the settling by the second ion exchange device 66 for the desalting processing, transporting the desalted raw material of the xylose mother liquid A to the vacuum crystallization assembly 67 for vacuum evaporation and concentration and vacuum boiling of sugar and crystallization to precipitate the crystal xylitol E, and obtaining the crystal xylitol E through a centrifugation operation and a drying operation.

The browning reaction processing of the browning reaction assembly 7 in the step three may include: obtaining a caramel pigment liquid F by performing a concentration processing, the browning reaction processing, and a filtering processing on the raffinate liquid D. A red index of the caramel pigment liquid F may be over 7, and an absorbance of the caramel pigment liquid F at 610 nm may be over 0.07.

The following is an embodiment that further illustrates the system and method for co-producing a xylitol and a caramel pigment by utilizing a xylose mother liquid of the present disclosure.

Embodiment 1:

The first embodiment of the system and method for co-producing a xylitol and a caramel pigment by utilizing a xylose mother liquid includes following steps.

In step one, a raw material of the xylose mother liquid A with a refraction of 60wt% in the raw material tank 1 was transported to the filter 2 through a pipeline for filtering impurities, and then the raw material of the xylose mother liquid A was transported to the nanofiltration membrane device 3 for a decolorizing processing. An operating temperature of the nanofiltration membrane device 3 was 45 °C and a pressure of the nanofiltration membrane device 3 was 30 bar.

In step two, the raw material of the xylose mother liquid A that is filtered and decolorized was transported to the first ion exchange device 4 for a desalting processing, and an electrical conductivity was controlled to be less than 50us/cm.

In step three, the ion exchange liquid B processed by the first ion exchange device 4 was transported to the chromatographic separation device 5 for a chromatographic separation processing, the extracted liquid C with a high xylose component content was obtained after the chromatographic separation processing, the extracted liquid C was transported for a refinement, crystallization, and a hydrogeneration reaction processing to obtain the crystal xylitol E with a purity over 99%, and the raffinate liquid D with a low xylose component content obtained after the chromatographic separation processing was transported to the browning reaction assembly 7 for a browning reaction processing. A yield rate of the desalting and decoloring process reached 95% and a yield rate of the crystal xylitol was 48%.

In step four, the extracted liquid C after evaporation and concentration was transported to the crystallization tank 62. The crystal xylitol was dissolved in water. A refraction was controlled at 60% and a pH value was at 5.00 to 7.00. A nickel catalyst with a mass percentage of 0.01% was added. A reaction temperature was controlled at 135°C and a steam pressure was above 0.4 MPa. The xylitol liquid with the nickel catalyst was transported to the hydrogenation reactor 65 for a hydrogenation reaction. A hydrogenation reaction pressure was controlled at 8 MPa, and a hydrogenation reaction time was 90 minutes; After the hydrogenation reaction, the reacted liquid was settled to remove the catalyst. The second ion exchange device 66 was used to desalt a hydrogenated liquid. The desalted liquid was transported to the vacuum crystallization assembly 67 to precipitate the crystal xylitol E. The crystal xylitol E with a purity of 99% was obtained through a centrifugation operation and a drying operation.

In step five, the raffinate liquid D was concentrated to a refraction of 80%, and a pH value was adjusted at 9.00. A compounded amino compound with a mass percentage of 9% (compounded by a urea and an ammonium carbonate with a compounding ratio of 1:2) was added as a catalyst. A reaction temperature of the browning reaction was controlled at 120°C and a time of the browning reaction was 240 minutes. The caramel pigment F liquid was obtained after the browning reaction. A color rate of the caramel pigment F liquid was 20000 EBC, a red index was 7.1, and an absorbance at 610 nm was 0.078.

A utilization rate of a reducing sugar in the extract liquid D reached 70% (calculated on a dry basis).

The utilization rate = a consumption of the reducing sugar in the browning reaction ÷ a total content of the reducing sugar in the extracted liquid D.

A price of the caramel pigment F prepared from the extracted liquid D may be at 3000 yuan per ton, and the value of the extracted liquid D is significantly increased.

Comparative Embodiment 1

A xylose mother liquid was directly utilized to prepare a xylitol, including steps as follows. A raw material of the xylose mother liquid A with a refraction of 60wt% in the raw material tank 1 was transported to the filter 2 through a pipeline for filtering impurities. 0.5% by weight of activated carbon was added to a decolorizing tank for a decolorizing processing. A plate and frame filter press was performed after the decolorizing processing. A filtrate liquid obtained was transported to the first ion exchange module 4 for a desalting processing. A yield rate of decolorizing and desalting processing was 85%. After a chromatographic separation, evaporation and concentration, and crystallization centrifugation operation, a yield rate of a crystal xylose was 45% (calculated on a dry basis). The xylitol was obtained after a hydrogenation processing was performed on the crystal xylose. A large amount of the raffinate liquid was effectively utilized and was ultimately processed as a mixed syrup. A selling price of the mixed syrup was 1500 yuan per ton. The value of the raffinate liquid was not increased.

In summary, the yield rate of the crystal xylitol increases from 45% to 48% through processes like nanofiltration and decolorization, ion exchange desalination. Through a high-value utilization of the raffinate liquid, the raffinate liquid with a selling price of 1500 yuan per ton can be transformed to the caramel pigment with a selling piece of 3000 yuan per ton, greatly enhancing the value of the xylose mother liquid.

The above descriptions are only exemplary embodiments of the present disclosure and should not be used to limit the present disclosure. Any modification, equivalent substitution, improvement made within the spirit and principle of the present disclosure should be included in the scope of protection of the present disclosure.

**Claims**

1.   A system for co-producing a xylitol and a caramel pigment by utilizing a xylitol mother liquid, comprising a raw material tank, a filter, a nanofiltration membrane device, a first ion exchange device, a chromatographic separation device, a refined hydrogenation assembly, and a browning reaction assembly that are sequentially interconnected through a pipeline, wherein

the raw material tank is configured to store the xylitol mother liquid,
the filter is configured to filter impurities in the xylitol mother liquid,
the nanofiltration membrane device is configured to decolorize the xylitol mother liquid that flows through the nanofiltration membrane device and obtain a retentate liquid and a permeation liquid, wherein the retentate liquid is a pigment liquid and the permeation liquid is a decolorized liquid,
the first ion exchange device is configured to desalt the decolorized liquid that flows through the first ion exchange device to obtain an ion exchange liquid,

the chromatographic separation device is configured to separate an extracted liquid with a high xylose component content and a raffinate liquid with a low xylose component content from the ion exchange liquid that flows through the chromatographic separation device,

the refined hydrogenation assembly is configured to prepare a crystal xylitol by performing a refined hydrogenation processing on the extracted liquid, and

the browning reaction assembly is configured to prepare the caramel pigment by performing a browning reaction processing on the raffinate liquid.

2. The system for co-producing a xylitol and a caramel pigment by utilizing a xylose mother liquid of claim 1, wherein the refined hydrogenation assembly includes: an evaporation and concentration device, a crystallization tank, a crystal xylose storage tank, a dissolving tank, a hydrogenation reactor, a second ion exchange device, and a vacuum crystallization assembly, wherein

the evaporation and concentration device is configured to further concentrate the extracted liquid,

the crystallization tank is configured to crystallize a xylose,

the crystal xylose storage tank is configured to store the crystal xylose,

the dissolving tank is configured to dissolve the crystal xylose into a xylose liquid and store the xylose liquid,

the hydrogenation reactor is configured to perform a hydrogenation and reduction reaction on the xylose liquid to generate the xylitol,

the second ion exchange device is configured to remove anions and cations in a xylitol liquid, and

the vacuum crystallization assembly is configured to perform a crystallization processing on the xylitol liquid processed by the second ion exchange device to obtain the crystal xylitol.

3. The system for co-producing a xylitol and a caramel pigment by utilizing a xylose mother liquid of claim 1, wherein the browning reaction assembly includes a concentration tank, a browning reaction reactor, and a browning reaction filter, wherein

the concentration tank is configured to concentrate the raffinate liquid to a certain concentration and store a concentrated raffinate liquid,

the browning reaction reactor is configured to prepare the caramel pigment by performing the browning reaction processing on the raffinate liquid, and

the browning reaction filter is configured to filter solid impurities in the caramel pigment.

4. A method for co-producing a xylitol and a caramel pigment by utilizing a xylose mother liquid, wherein the method is based on a system for co-producing a xylitol and a caramel pigment by utilizing a xylose mother liquid according to any one of claims 1 to 3, the method comprising:

in step one, transporting a raw material of the xylose mother liquid in a raw material tank to a filter through a pipeline for filtering impurities, and then transporting the raw material of the xylose mother liquid that is filtered to a nanofiltration membrane device for a decolorizing processing;

in step two, transporting the raw material of the xylose mother liquid that is filtered and decolorized to a first ion exchange device for a desalting processing and obtaining an ion exchange liquid; and

in step three, transporting the ion exchange liquid to a chromatographic separation device for a chromatographic separation processing, obtaining an extracted liquid with a high xylose component content and a raffinate liquid with a low xylose component content after the chromatographic separation processing, transporting the extracted liquid to a refined hydrogenation assembly for a refined hydrogenation processing to obtain a crystal xylitol with a purity over 99%, and transporting the raffinate liquid to a browning reaction assembly for a browning reaction processing to prepare the caramel pigment.

5. The method for co-producing a xylitol and a caramel pigment by utilizing a xylose mother liquid of claim 4, wherein in the step one, a mass percentage concentration of a dry matter of the xylose mother liquid is 50 to 60wt%, the mass percentage concentration being a sugar concentration, and in the dry matter, a content of glucose is 12 to 18wt%, a content of xylose is 40 to 50wt%, a content of arabinose is 17 to 23wt%, a content of mannose is 10 to 22wt%, and a content of galactose is 0 to 6wt%.

6. The system for co-producing a xylitol and a caramel pigment by utilizing a xylitol mother liquid of claim 4, wherein in the step one, during the decolorizing processing, an operating temperature of the nanofiltration membrane device is 40 °C to 48 °C, an operating pressure of the nanofiltration membrane device is 25 bar to 35 bar, and a yield rate reaches 90% to 98%.

7. The method for co-producing a xylitol and a caramel pigment by utilizing a xylitol mother liquid of claim 4, further comprising: in the step two, during the desalting processing, controlling an electrical conductivity rate being smaller than 50 us/cm, a yield rate reaching 90% to 98 %.

8. The method for co-producing a xylitol and a caramel pigment by utilizing a xylitol mother liquid of claim 4, further comprising: in the step three, dissolving the xylose in water, controlling a refraction being between 50% to 60% and a pH value being 5.00 to 7.00, adding a nickel catalyst with a mass percentage of 0.01% to 0.02%, controlling a reaction temperature being between 130°C to 140°C and a steam pressure being above 0.4 MPa, controlling a hydrogenation reaction pressure being between 7.0 MPa to 9.5 MPa and a hydrogenation reaction time being 60 minutes to 120 minutes; concentrating the raffinate liquid to a refraction between 75% to 85%, adjusting a pH value being between 7.00 to 9.00, adding a compounded amino compound with a mass percentage of 6% to 12% as a catalyst, and controlling a reaction temperature of the browning reaction being between 120°C to 140 °C and a time of the browning reaction being 60 minutes to 240 minutes.

9. The method for co-producing a xylitol and a caramel pigment by utilizing a xylose mother liquid of claim 4, wherein the refined hydrogenation processing of the refined hydrogenation assembly in the step three includes: transporting the extracted liquid after evaporation and concentration to the crystallization tank, dissolving the crystal xylose in water, transporting the xylose liquid into the hydrogenation reactor for a hydrogenation reaction to obtain a xylitol solution, settling the xylitol solution after the hydrogenation reaction is completed to remove the catalyst, performing the desalting processing on a supernatant liquid of the settling by the second ion exchange device, transporting the desalted liquid to the vacuum crystallization assembly for vacuum evaporation and concentration and vacuum boiling of sugar and crystallization to precipitate the crystal xylitol, and obtaining the crystal xylitol through a centrifugation operation and a drying operation.

10. The method for co-producing a xylitol and a caramel pigment by utilizing a xylose mother liquid of claim 4, wherein the browning reaction processing by the browning reaction assembly in the step three includes: obtaining a caramel pigment liquid by performing a concentration processing, the browning reaction processing, and a filtering processing on the raffinate liquid, a red index of the caramel pigment liquid being over 7, and an absorbance of the caramel pigment liquid at 610 nm being over 0.07

Fig 1

Fig 2

## INTERNATIONAL SEARCH REPORT

| International application No. |
| --- |
| **PCT/CN2022/125220** |

**A. CLASSIFICATION OF SUBJECT MATTER**

C07C 31/18(2006.01)i;  C07C 29/78(2006.01)i;  C07C 29/76(2006.01)i;  C09B 61/00(2006.01)i;  B01J 19/00(2006.01)i

According to International Patent Classification (IPC) or to both national classification and IPC

**B. FIELDS SEARCHED**

Minimum documentation searched (classification system followed by classification symbols)

C07C; C09B; B01J

Documentation searched other than minimum documentation to the extent that such documents are included in the fields searched

Electronic data base consulted during the international search (name of data base and, where practicable, search terms used)

CNTXT; VEN; WOTXT; USTXT; EPTXT; CNKI; 万方, WANFANG; STN: 浙江华康药业, 李勉, 徐伟冬, 陈德水, 程新平, 廖承军, 吴强, 杨武龙, 秦淑芳, 木糖, 母液, 木糖醇, 焦糖色素, 过滤, 纳滤膜, 离子交换, 色谱分离, 氢化, 脱色, 脱盐, xylitol, xylose, mother liquor, filter, nanofilt+, Caramel+, Ion exchange+, Chromatograp+, hydrogen+, decoloriz+, desalinat+

**C. DOCUMENTS CONSIDERED TO BE RELEVANT**

| Category* | Citation of document, with indication, where appropriate, of the relevant passages | Relevant to claim No. |
| --- | --- | --- |
| PX | CN 114213215 A (ZHEJIANG HUAKANG PHARMACEUTICAL CO., LTD.) 22 March 2022 (2022-03-22)<br>claims 1-10 | 1-10 |
| Y | CN 109503676 A (ZHEJIANG HUAKANG PHARMACEUTICAL CO., LTD. et al.) 22 March 2019 (2019-03-22)<br>claim 1, and description, paragraph [0006] | 1-10 |
| Y | CN 113214531 A (JIANGNAN UNIVERSITY) 06 August 2021 (2021-08-06)<br>description, paragraphs [0004]-[0006] | 1-10 |
| Y | CN 103409315 A (CHONGQING UNIVERSITY) 27 November 2013 (2013-11-27)<br>claim 1, and description, paragraph [0049] | 1-10 |
| Y | CN 107893132 A (JIANGSU JIUWU HI-TECH CO., LTD.) 10 April 2018 (2018-04-10)<br>claim 1 | 1-10 |
| A | CN 101823939 A (SHANDONG FUTIAN PHARMACEUTICAL CO., LTD.) 08 September 2010 (2010-09-08)<br>entire document | 1-10 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **12 December 2022** | **12 January 2023** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2022/125220** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 101857523 A (YUCHENG LUJIAN BIOTECHNOLOGY CO., LTD.) 13 October 2010 (2010-10-13)<br>    entire document | 1-10 |
| A | CN 102241707 A (ZHEJIANG HUAKANG PHARMACEUTICAL CO., LTD.) 16 November 2011 (2011-11-16)<br>    entire document | 1-10 |
| A | CN 106591384 A (ZHEJIANG HUAKANG PHARMACEUTICAL CO., LTD.) 26 April 2017 (2017-04-26)<br>    entire document | 1-10 |

Form PCT/ISA/210 (second sheet) (January 2015)

INTERNATIONAL SEARCH REPORT
Information on patent family members

International application No.

**PCT/CN2022/125220**

| Patent document cited in search report | | | Publication date (day/month/year) | Patent family member(s) | | | Publication date (day/month/year) |
|---|---|---|---|---|---|---|---|
| CN | 114213215 | A | 22 March 2022 | CN | 216614473 | U | 27 May 2022 |
| CN | 109503676 | A | 22 March 2019 | CN | 109503676 | B | 11 September 2020 |
| CN | 113214531 | A | 06 August 2021 | CN | 113214531 | B | 19 August 2022 |
| CN | 103409315 | A | 27 November 2013 | | None | | |
| CN | 107893132 | A | 10 April 2018 | | None | | |
| CN | 101823939 | A | 08 September 2010 | CN | 101823939 | B | 02 January 2013 |
| CN | 101857523 | A | 13 October 2010 | CN | 101857523 | B | 03 April 2013 |
| CN | 102241707 | A | 16 November 2011 | | None | | |
| CN | 106591384 | A | 26 April 2017 | | None | | |

Form PCT/ISA/210 (patent family annex) (January 2015)

## REFERENCES CITED IN THE DESCRIPTION

**Patent documents cited in the description**

- CN 109503676 A